# EUROPÄISCHE PATENTANMELDUNG

(11) **EP 2 168 498 A1**
(43) Veröffentlichungstag der Anmeldung: **31.03.2010**
(21) Anmeldenummer: 09169621.1
(22) Anmeldetag: 07.09.2009
(51) Int. Cl.: A61B 10/00

(54) **Vorrichtung und Verfahren zum Entnehmen von Material eines Material-Reservoirs**

(30) Priorität: 25.09.2008 DE 102008048866
(71) Anmelder: Siemens Aktiengesellschaft, 80333 München (DE)
(72) Erfinder: Eckstein, Gerald, 80995, München (DE); Hiltawsky, Karsten, 58239, Schwerte (DE); Schneider, Heinz-Ingo, 85598, Baldham (DE); Sickert, Daniel, 81679, München (DE)

(57) **Zusammenfassung**

Die Erfindung betrifft eine Vorrichtung (10) zum Entnehmen von Material eines Material-Reservoirs, aufweisend mindestens ein Führungsrohr (2) mit Innenraum und mit einer den Innenraum begrenzenden Innenwandung, mindestens einen Schlitten, der im Innenraum entlang einer Längsausrichtung des Führungsrohres eine Schlitten-Translations-Bewegung durchführen kann, und mindestens eine Sammel-Einheit (4) mit Sammel-Oberfläche (40) zum Sammeln des Materials, wobei die Sammel-Einheit und der Schlitten derart aneinander gekoppelt sind, dass die Schlitten-Translations-Bewegung des Schlittens zu einer Sammel-Translations-Bewegung der Sammel-Oberfläche der Sammel-Einheit führt und dass so die Sammel-Oberfläche in das Material-Reservoir eingeführt und aus dem Material-Reservoir herausgeführt werden kann. Durch geeignete Maßnahmen wird ein manuell oder automatisiert zu betätigender Drehmechanismus realisiert, über den ein Draht, der als Sammel-Einrichtung fungiert, über eine Länge von 1 mm bis zu 2 cm innerhalb einer Venen-Verweil-Kanüle verschoben werden kann. Anwendung findet die Erfindung beispielsweise in der Medizintechnik zum Sammeln von Zellen aus einem Reservoir in Form einer Körperflüssigkeit.

## Beschreibung

Die Erfindung betrifft eine Vorrichtung und ein Verfahren zum Entnehmen von Material eines Material-Reservoirs.

Zum Sammeln von Probenmaterial im Blutkreislauf als Material-Reservoir können Sammel-Einrichtungen in Form von dünnen Drähten in eine in einem Blutgefäß sitzende Kanüle eingeführt werden. An der Draht-Spitze des dünnen Drahts lagert sich das Material ab. Nach dem Sammeln des Materials muss der gesamte Draht durch die gesamte Kanüle gezogen werden. Dabei berührt der Draht eine Außenwand der Kanülenröhre, wobei es zu einer abrasiven Belastung an der Drahtoberfläche kommt und das gesammelte Material abgestreift oder verändert werden kann.

Aufgabe der vorliegenden Erfindung ist es, eine Möglichkeit anzugeben, mit der die geschilderte abrasive Belastung vermieden werden kann.

Zur Lösung der Aufgabe wird eine Vorrichtung zum Entnehmen von Material eines Material-Reservoirs angegeben, aufweisend mindestens ein Führungsrohr mit Innenraum und mit einer den Innenraum begrenzenden Innenwandung, mindestens einen Schlitten, der im Innenraum entlang einer Längsausrichtung des Führungsrohres eine Schlitten-Translations-Bewegung durchführen kann, und mindestens eine Sammel-Einheit mit Sammel-Oberfläche zum Sammeln des Materials, wobei die Sammel-Einheit und der Schlitten derart aneinander gekoppelt sind, dass die Schlitten-Translations-Bewegung des Schlittens zu einer Sammel-Translations-Bewegung der Sammel-Oberfläche der Sammel-Einheit führt und dass so die Sammel-Oberfläche in das Material-Reservoir eingeführt und aus dem Material-Reservoir herausgeführt werden kann.

Zur Lösung der Aufgabe wird auch ein Verfahren zum Entnehmen von Material eines Material-Reservoirs unter der Verwendung der Vorrichtung mit folgendem Verfahrensschritt angegeben: Zusammenbringen der Sammel-Oberfläche der Sammel-Einrichtung durch Ausführen der Schlitten-Translations-Bewegung des Schlittens, wobei durch das Zusammenbringen ein Anlagern des Materials des Material-Reservoirs an der Sammel-Oberfläche ermöglicht wird.

Die grundlegende Idee der Erfindung besteht darin, mit Hilfe des Führungsrohrs und dem Schlitten, ein definiertes Einführen der Sammel-Oberfläche in das Material-Reservoir sicher zu stellen. Es wird gewährleistet, dass es weder beim Einführen der Sammel-Oberfläche in das Material-Reservoir noch beim Entfernen der Sammel-Oberfläche aus dem Material-Reservoir zu abrasiven Belastungen der Sammel-Oberfläche kommt, beispielsweise dadurch, dass die Sammel-Oberfläche unkontrolliert an eine Kanülen-Wandung der Kanüle gelangt.

Gemäß einer besonderen Ausgestaltung weist die Innenwandung des Führungsrohres ein entlang der Längsausrichtung des Führungsrohres ausgerichtetes Führungslager zum Führen eines mit dem Schlitten verbundenen Zapfens auf. Das Führungslager ist eine Nut, in die der Zapfen (Dorn) des Schlittens hineinragt.

Gemäß einer weiteren Ausgestaltung verläuft das Führungslager im Wesentlichen parallel zur Längsausrichtung des Führungsrohres. Im Wesentlichen bedeutet in diesem Zusammenhang, dass Abweichungen von bis zu 10° von der exakten parallelen Ausrichtung auftreten können.

In einer besonderen Ausgestaltung ist das Führungslager gegen die Längsausrichtung des Führungsrohres verkippt. Das Führungslager und die Längsausrichtung sind in einem Winkel zu einander angeordnet. Es resultiert eine Steigung beziehungsweise ein Steigungswinkel. Bei fixiertem, also fest stehendem Führungsrohr kommt es durch das Führen des Zapfens im Führungslager zusätzlich zur Schlitten-Translations-Bewegung zu einer Schlitten-Rotations-Bewegung des Schlittens. Sind die Sammel-Einheit mit der Sammel-Oberfläche und der Schlitten fest miteinander verbunden, so resultiert aus der Schlitten-Rotations-Bewegung eine Sammel-Rotations-Bewegung.

Eine bevorzugte Lösung für die beschriebene Vorrichtung sieht wie folgt aus: Das Führungsrohr ist als Hohlzylinder und der Schlitten als Kolben ausgestaltet. Dabei kann der Kolben formschlüssig im Hohlzylinder bewegt werden. Kolben und Hohlzylinder bilden einen Formschluss.

In einer besonderen Ausgestaltung ist im Innenraum des Führungsrohres eine mit dem Führungsrohr Führungsschiene zum Führen des Schlittens vorhanden, so dass der Schlitten im Wesentlichen nur die Schlitten-Translations-Bewegung durchführen kann. Die Bewegung des Schlittens ist von einer möglichen Bewegung des Führungsrohres entkoppelt. Durch diese Lösung ist gewährleistet, dass lediglich die Schlitten-Translations-Bewegung stattfindet. Wenn die Sammel-Einheit fest mit dem Schlitten verbunden ist, findet auch nur eine Sammel-Translations-Bewegung statt. Die Sammel-Oberfläche dreht sich nicht. Es resultiert eine zusätzlich verminderte abrasive Belastung der Sammel-Oberfläche.

Gemäß einer besonderen Ausgestaltung ist eine Hubeinrichtung zum Einstellen eines Schlitten-Hubs des Schlittens entlang der Längsausrichtung des Führungsrohres vorhanden. Die Hubeinrichtung weist beispielsweise einen Stellantrieb in Form eines Motors auf. Durch Ansteuerung des Stellantriebs wird die Stellung (der Schlitten-Hub) im Innenraum des Führungsrohres eingestellt.

Insbesondere weist die Hub-Einrichtung das Führungsrohr mit dem Führungslager auf. Das Führungsrohr ist derart drehbar ausgeführt, dass eine Drehung des Führungsrohres zur Schlitten-Translations-Bewegung führt. Dies ist beispielsweise dann möglich, wenn der Schlitten, wie oben beschrieben, die Schlitten-Translations-Bewegung in einer fest mit dem Führungsrohr verbundenen Führungsschiene vollziehen kann. Es resultiert ein einfacher Drehmechanismus, bei dem die Drehung des Führungsrohres und die Schlitten-Translations-Bewegung entkoppelt sind. Durch die Drehung wird der Schlitten-Hub eingestellt. Wenn die Sammel-Oberfläche und der Schlitten fest miteinander verbunden sind, findet keine Sammel-Rotations-Bewegung, sondern lediglich die Sammel-Translations-Bewegung statt. Ein Sammel-Hub der Sammel-Oberfläche und damit ein Hineinragen der Sammel-Oberfläche in das Material-Reservoir kann leicht eingestellt werden.

Gemäß einer weiteren Ausgestaltung umfasst das Führungslager mindestens eine Ausnehmung des Führungslagers zum Einrasten des Zapfens. Durch das Einrasten des Zapfens in der Ausnehmung kann der Schlitten-Hub stufenweise verstellt werden. Zum Einrasten ist es vorteilhaft, wenn der Zapfen gebogen werden kann.

Für das Sammeln der Probe ist die Sammel-Oberfläche entsprechend ausgestaltet. In einer besonderen Ausgestaltung ist die Sammel-Oberfläche von einer Beschichtung der Sammel-Einheit gebildet. Die Vorrichtung lässt sich auf diese Weise auf verschiedenste Anwendungsgebiete hin modifizieren. Allein durch die Beschichtung wird festgelegt, welches Material sich an der Oberfläche anlagert. Das Material kann ein anorganisches oder organisches Material sein. Denkbar sind insbesondere biologische Materialien in Form von Zellen oder Proteinen.

In einer besonderen Ausgestaltung weist die Sammel-Einheit einen Draht auf. Dabei ist die Sammel-Oberfläche von einer Draht-Oberfläche des Drahts gebildet. Beispielsweise befindet sich die Sammel-Oberfläche an einer Draht-Spitze des Drahtes. Mit Hilfe der Vorrichtung wird die Draht-Spitze in das Material-Reservoir geführt.

Um die abrasive Belastung der Drahtoberfläche beziehungsweise der Sammel-Oberfläche weiter zu vermindern, ist es besonders vorteilhaft, zusätzlich eine Schutzhülle vorzusehen. Der Draht beziehungsweise die Drahtoberfläche wird aus der Schutzhülle in das Material-Reservoir eingefahren bzw. aus dem Material-Reservoir in die Schutzhülle eingefahren. Die Schutzhülle ist beispielsweise eine Kanüle. Die Schutzhülle ist dabei beispielsweise formschlüssig mit dem Führungsrohr verbunden. Dazu ist bevorzugt ein entsprechend angepasster Adapter vorhanden. Im Fall eines Material-Reservoirs in Form von Blut wird insbesondere ein Adapter in Form eines Luer-Lock-Anschlusses eingesetzt.

Die Vorrichtung kann zur Entnahme von Material aus einem Material-Reservoir auf den verschiedensten Gebieten angewendet werden. Durch die Entnahme kann beispielsweise eine Qualität des Material-Reservoirs überprüft werden. Die Qualität hängt beispielsweise von einer Zusammensetzung des Material-Reservoirs ab. Alterungsprozesse des Material-Reservoirs sind beispielsweise über Zersetzungsprodukte im Material-Reservoir nachweisbar. Ebenso können Verunreinigungen mit Hilfe der Vorrichtung nachgewiesen werden. Besonders einfach gelingt der Nachweis, wenn die Sammel-Oberfläche Bestandteil eines entsprechenden Sensors ausgestaltet ist.

Als Material-Reservoir kommen beliebige Fluide (Flüssigkeiten, Gase, Mischungen von Gasen und Flüssigkeiten) in Frage. So reicht das Anwendungsgebiet von Lebensmitteln (z.B. Wasser) bis hin zu organischen Lösungsmitteln und zu Schmierstoffen und Ölen (z.B. Motoröl). Insbesondere wird als Material-Reservoir ein Material-Reservoir eines Organismus verwendet. Das Material-Reservoir eines Organismus ist beispielsweise ebenfalls ein Fluid in Form einer Körperflüssigkeit des Organismus. Die Körperflüssigkeit ist beispielsweise Blut, Urin, Zellflüssigkeit, etc. Im Hinblick auf einen Organismus kann das Material-Reservoir auch eine Ansammlung von Zellen sein, aus der beispielsweise über eine Punktion Material entnommen werden soll. In diesem Fall wäre das Material-Reservoir nicht fluidischer Natur.

Das zu sammelnde Material kann lediglich aus kleinen Molekülen bestehen. Denkbar sind insbesondere Materialien in Form von großen, komplexen biologischen Systemen, beispielsweise in Form von Proteinen oder Zellen.

Folgende Vorteile der Erfindung sind hervorzuheben:
- Mit der Erfindung ist es möglich, Material an einer Sammel-Oberfläche einer Sammel-Einheit ohne erhebliche abrasive Belastung zu sammeln und zu entnehmen.
- Durch den vorgeschlagenen Drehmechanismus kann die Sammel-Oberfläche, z.B. in Form eines Drahts, um eine definierte Länge verschoben werden. Dabei dreht sich gemäß einer besonderen Ausgestaltung die Sammel-Oberfläche nicht, was zu einer weiter verminderten abrasiven Belastung der Sammel-Oberfläche führt.
- Die Sammel-Oberfläche in Form einer von einer Draht-Spitze eines Drahts gebildeten Draht-Oberfläche muss nicht vollständig durch die entsprechende Kanüle gezogen werden.

Anhand eines Ausführungsbeispiels und der dazugehörigen Figuren wird die Erfindung im Folgenden näher beschrieben. Die Figuren sind schematisch und stellen keine maßstabsgetreuen Abbildungen dar.

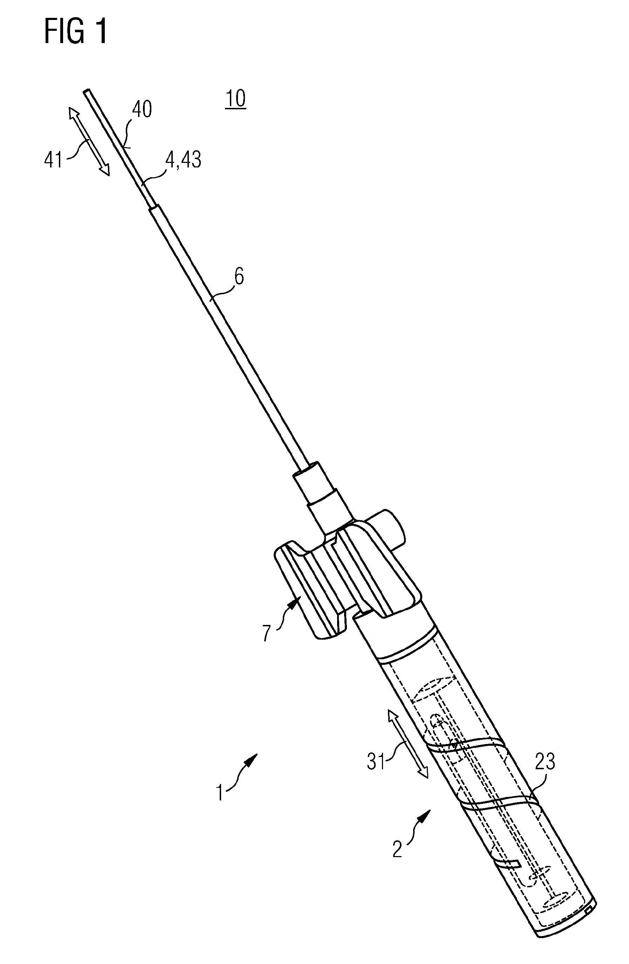

- Figur 1: zeigt eine Vorrichtung zum Entnehmen von Material eines Material-Reservoirs in perspektivischer Darstellung.

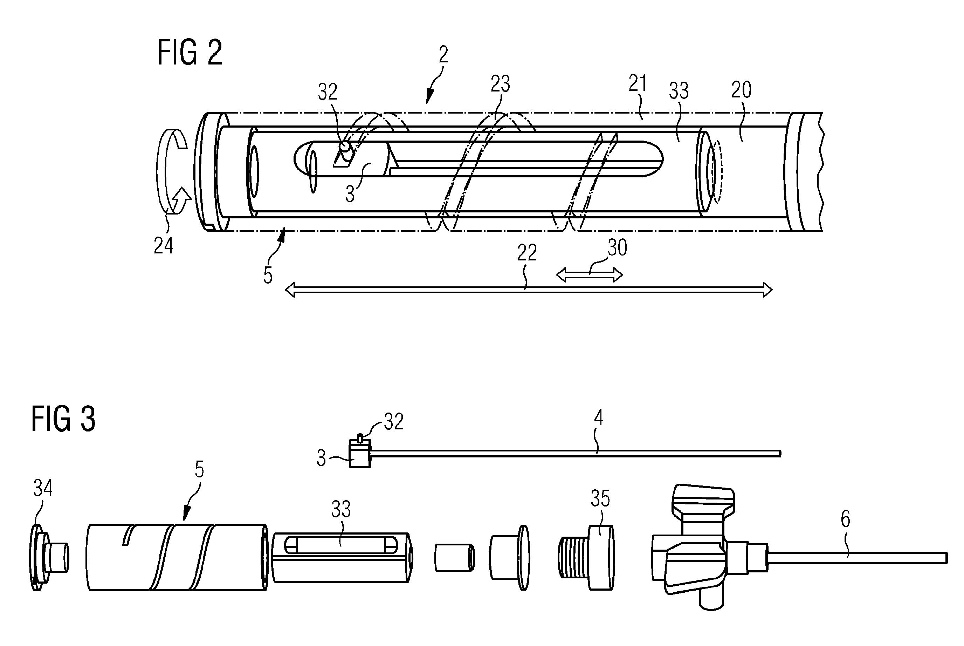

- Figur 2: zeigt einen Ausschnitt der Vorrichtung zum Entnehmen von Material in perspektivischer Darstellung.
- Figur 3: zeigt eine Explosionszeichnung der Vorrichtung zum Entnehmen von Material eines Material-Reservoirs.

Die Vorrichtung 1 zum Entnehmen von Material eines Material-Reservoirs 10 weist ein Führungsrohr 2 mit Innenraum 20 und mit einer den Innenraum begrenzenden Innenwandung 21 auf. Innerhalb des Innenraums des Führungsrohrs ist ein Schlitten 3 derart angeordnet, dass er entlang einer Längsausrichtung 22 des Führungsrohres eine Schlitten-Translations-Bewegung 30 durchführen kann.

Darüber hinaus ist eine Sammel-Einheit 4 mit Sammel-Oberfläche 40 zum Sammeln des Materials vorhanden. Die Sammel-Einheit und der Schlitten sind derart aneinander gekoppelt, dass die Schlitten-Translations-Bewegung des Schlittens zu einer Sammel-Translations-Bewegung 41 der Sammel-Oberfläche der Sammel-Einheit führt und dass so die Sammel-Oberfläche in das Reservoir eingeführt und aus dem Reservoir herausgeführt werden kann. Die Sammel-Einheit wird von einem Draht und die Sammel-Oberfläche von einer Draht-Oberfläche am Ende des Drahts.

Die Innenwandung des Führungsrohres weist ein entlang der Längsausrichtung des Führungsrohres ausgerichtetes Führungslager (Nut) 23 zum Führen eines mit dem Schlitten verbundenen Zapfens 32 auf. Das Führungslager ist gegen die Längsausrichtung des Führungsrohres verkippt ist.

Im Innenraum des Führungsrohres ist eine Führungsschiene 33 zum Führen des Schlittens vorhanden, so dass der Schlitten im Wesentlichen nur die Schlitten-Translations-Bewegung durchführen kann. Eine Schlitten-Rotations-Bewegung wird durch die Schlitten-Translations-Bewegung nicht initiiert. Innerhalb der Führungsschiene erfolgt der Schlitten-Hub 31 des Schlittens. Zum festen Verbinden der Führungsschiene mit dem Führungsrohr ist die Führungsschiene mit den Schrauben 34 und 35 in das Führungsrohr eingespannt.

Zum Einstellen des Schlitten-Hubs des Schlittens entlang der Längsausrichtung des Führungsrohres ist eine Hub-Einrichtung 5 vorhanden ist. Die Hub-Einrichtung weist das Führungsrohr mit dem Führungslager auf. Dabei ist das Führungsrohr derart drehbar ausgeführt ist, dass eine Drehung 24 des Führungsrohres zur Schlitten-Translations-Bewegung führt.

Gemäß weiterer, nicht dargestellter Ausführungsformen weist die Führungsschiene Ausnehmungen auf, in die der Zapfen einrasten kann. Dazu kann der Zapfen gebogen werden. Alternativ dazu wird die Schiene federnd gelagert. Dazu weisen die Schrauben 34 und 35 nicht dargestellte Federelemente auf.

Gemäß dem Ausführungsbeispiel ist das Material-Reservoir Blut, das in einer Vene verläuft. Innerhalb einer Schutzhülle 6 in Form einer Venenkanüle, die mit einen Luer-Lock-Anschluss 7 formschlüssig verbunden ist, kann der Draht linear bewegt werden. Ein maximaler Hub des Drahts ist so dimensioniert, dass das freie Drahtende mit der Sammel-Oberfläche einen für die Anwendung definierten Überstand zur Venenkanüle aufweist. Dieser Überstand, mit dem der Draht in die Vene hineinragen kann, beträgt bis zu 30 mm. Bei minimalem Hub taucht die Draht-Spitze in die Venenkanüle ein. Die Draht-Spitze verschwindet in der Venenkanüle. Aufgrund des Drehmechanismus kann der Draht innerhalb der Venenkanüle über eine Länge von 1 mm bis zu 2 cm verschoben werden.

## Patentansprüche

1. Vorrichtung (1) zum Entnehmen von Material eines Material-Reservoirs, aufweisend
- mindestens ein Führungsrohr (2) mit Innenraum (20) und mit einer den Innenraum begrenzenden Innenwandung (21),
- mindestens einen Schlitten (3), der im Innenraum entlang einer Längsausrichtung (22) des Führungsrohres eine Schlitten-Translations-Bewegung (30) durchführen kann, und
- mindestens eine Sammel-Einheit (4) mit Sammel-Oberfläche (40) zum Sammeln des Materials, wobei
- die Sammel-Einheit und der Schlitten derart aneinander gekoppelt sind, dass die Schlitten-Translations-Bewegung des Schlittens zu einer Sammel-Translations-Bewegung (41) der Sammel-Oberfläche der Sammel-Einheit führt und dass so die Sammel-Oberfläche in das Material-Reservoir eingeführt und aus dem Material-Reservoir herausgeführt werden kann.

2. Vorrichtung nach Anspruch 1, wobei die Innenwandung des Führungsrohres ein entlang der Längsausrichtung des Führungsrohres ausgerichtetes Führungslager (23) zum Führen eines mit dem Schlitten verbundenen Zapfens (32) aufweist.

3. Vorrichtung nach Anspruch 2, wobei das Führungslager im Wesentlichen parallel zur Längsrichtung des Führungsrohres verläuft.

4. Vorrichtung nach Anspruch 2, wobei das Führungslager gegen die Längsausrichtung des Führungsrohres verkippt ist.

5. Vorrichtung nach einem der Ansprüche 2 bis 4, wobei im Innenraum des Führungsrohres eine Führungsschiene (33) zum Führen des Schlittens vorhanden ist, so dass der Schlitten im Wesentlichen nur die Schlitten-Translations-Bewegung durchführen kann.

6. Vorrichtung nach einem der Ansprüche 1 bis 5, wobei eine Hub-Einrichtung zum Einstellen eines Schlitten-Hubs des Schlittens entlang der Längsausrichtung des Führungsrohres vorhanden ist.

7. Vorrichtung nach Anspruch 6, wobei die Hub-Einrichtung das Führungsrohr mit dem Führungslager aufweist und das Führungsrohr derart drehbar ausgeführt ist, dass eine Drehung des Führungsrohres zur Schlitten-Translations-Bewegung führt.

8. Vorrichtung nach einem der Ansprüche 2 bis 7, wobei das Führungslager mindestens eine Ausnehmung des Führungslagers zum Einrasten des Zapfens umfasst.

9. Vorrichtung nach einem der Ansprüche 1 bis 8, wobei die Sammel-Oberfläche von einer Beschichtung der Sammel-Einheit gebildet ist.

10. Vorrichtung nach einem der Ansprüche 1 bis 9, wobei die Sammel-Einheit einen Draht aufweist und die Sammel-Oberfläche von einer Draht-Oberfläche des Drahts gebildet ist.

11. Vorrichtung nach einem der Ansprüche 1 bis 10, wobei eine Schutzhülle zur Aufnahme der Sammel-Einheit vorhanden ist.

12. Vorrichtung nach Anspruch 11, wobei die Schutzhülle von einer Kanüle gebildet ist.

13. Verfahren zum Entnehmen von Material eines Material-Reservoirs unter der Verwendung der Vorrichtung nach einem der Ansprüche 1 bis 12 mit folgendem Verfahrensschritt: Zusammenbringen der Sammel-Oberfläche der Sammel-Einrichtung durch Ausführen der Schlitten-Translations-Bewegung des Schlittens, wobei durch das Zusammenbringen ein Anlagern des Materials des Material-Reservoirs an der Sammel-Oberfläche ermöglicht wird.

14. Verfahren nach Anspruch 13, wobei als Material-Reservoir ein Fluid verwendet wird.

15. Verfahren nach Anspruch 13 oder 14, wobei als Material-Reservoir ein Material-Reservoir eines Organismus verwendet wird.

16. Verfahren nach Anspruch 15, wobei das Material biologisches Material ist.
